# EUROPÄISCHE PATENTSCHRIFT

(11) **EP 1 787 570 B1**
(45) Veröffentlichungstag und Bekanntmachung des Hinweises auf die Patenterteilung: **29.12.2010**
(21) Anmeldenummer: 05025021.6
(22) Anmeldetag: 16.11.2005
(51) Int. Cl.: A61B 1/00

(54) **Optisches Instrument**
Optical instrument
Instrument optique

(43) Veröffentlichungstag der Anmeldung: 23.05.2007
(73) Patentinhaber: Richard Wolf GmbH, 75438 Knittlingen (DE)
(72) Erfinder: Weigel, Martin, Dipl.-Ing., 75433 Maulbronn (DE); Frank, Alexander, 75038 Oberderdingen (DE)
(74) Vertreter: Vollmann, Heiko

(56) Entgegenhaltungen:
- EP-A- 0 592 194
- US-A- 5 621 830
- US-A- 5 797 836
- US-A- 5 817 014

## Beschreibung

Die Erfindung betrifft ein optisches Instrument, insbesondere ein Endoskop oder Technoskop mit den im Oberbegriff des Anspruchs 1 angegebenen Merkmalen.

Bei derartigen Instrumenten, die typischerweise mit einer Seitblickoptik ausgestattet sind, d. h. ein Optikausblickfenster aufweisen, das in einem Winkel zur Längsachse von beispielsweise 30° angeordnet ist, zählt es zum Stand der Technik, den optoelektrischen Wandler, typischerweise ein im Schaft angeordnetes CCD-Element drehbar anzuordnen, und zwar so, dass die Bedienperson typischerweise vom Handhabenteil aus mit einem dort befindlichen Drehrad oder dergleichen den Drehwinkel des CCD-Wandlers zum Instrument verändern kann. Dies ist insbesondere dazu vorgesehen, um nach dem Drehen des Instrumentes um seine Längsachse das auf dem Monitor angezeigte Bild wieder aufrechtstehend ausrichten zu können. Ein solches Instrument ist beispielsweise aus US-PS 5,797,836 bekannt.

Da derartige optische Instrumente, wenn sie beispielsweise in der Medizin als Endoskop eingesetzt werden, hohen Temperaturen im Sterilisator ausgesetzt sind oder, wenn es sich z. B. um Technoskope handelt, auch hohe Temperaturen im Betrieb auftreten können, ist es einerseits erforderlich, das gesamte Instrument mit der darin befindlichen Optik und auch etwaigen beweglichen Teilen vollständig zu kapseln, andererseits jedoch bei den beweglichen Teilen ein ausreichendes Spiel vorzusehen, damit die aufgrund von Temperaturschwankungen auftretenden Längenänderungen der Bauteile aufgenommen werden können. Ein axiales Spiel zwischen CCD-Element und endseitigem Ausblickfenster kann jedoch dazu führen, dass die Abbildungsschärfe ungünstig beeinflusst wird. Es zählt daher auch zum Stand der Technik, bei derartigen optischen Instrumenten Mittel zum axialen Verstellen des CCD-Elementes im Bezug auf die Optik vorzusehen. Solche Verstellmittel sind entweder als manuell zu betätigende Justagemittel am Instrument (US 5,797,836) oder als rechnergestütztes Regelsystem (US 5,817,014) realisiert. Dies ist jedoch insbesondere unter Berücksichtigung, dass das Instrument nach Möglichkeit hermetisch abgeschlossen sein soll, konstruktiv sehr aufwändig, kostenintensiv und auch störanfällig.

Vor diesem Hintergrund liegt der Erfindung die Aufgabe zugrunde, ein optisches Instrument der Eingangs genannten Art so auszubilden, dass einerseits eine Drehbarkeit des optoelektrischen Wandlers im Bezug auf das Instrument möglich ist, andererseits jedoch in Richtung der Längsachse des Instrumentes stets die richtige Position des optoelektrischen Wandlers im Bezug auf die Optik gewährleistet ist, ohne dass eine manuelle Einstellung am Instrument oder ein rechnergestütztes Regelsystemsystem erforderlich ist.

Diese Aufgabe wird gemäß der Erfindung durch ein optisches Instrument mit den im Anspruch 1 angegebenen Merkmalen gelöst. Vorteilhafte Ausgestaltungen der Erfindung ergeben sich aus den Unteransprüchen, der nachfolgenden Beschreibung und der Zeichnung.

Das erfindungsgemäße optische Instrument, bei dem es sich typischerweise um ein Endoskop oder ein Technoskop handelt, weist einen Schaft auf, an dessen oder nahe dessen freien Schaftende ein Ausblickfenster vorgesehen ist, wobei dieser Schaft mit seinem anderen Ende in einem Handhabenteil festgelegt ist. Innerhalb des Schaftes ist ein optoelektrischer Wandler, beispielsweise ein CCD-Element angeordnet. Dieser Wandler ist drehbar innerhalb des Schaftes angeordnet und in dem er innerhalb eines drehbar im Schaft gelagerten Innenschaft angebracht ist. Zum Drehen des Wandlers im Schaft sind Betätigungsmittel, typischerweise am Handhabenteil vorgesehen. Zwischen Schaft und Innenschaft, welcher den Wandler trägt, ist gemäß der findung ein Axiallager vorgesehen, darüber hinaus ist mindestens ein den Innenschaft in Richtung zum Axiallager kraftbeaufschlagendes Federelement vorgesehen. Diese erfindungsgemäße Lösung sorgt dafür, dass der Wandler zusammen mit dem Innenschaft, in dem er festgelegt ist, zwar in Achsrichtung des Schaftes mit einem vergleichsweise großen Spiel beweglich geführt sein kann, jedoch aufgrund des Federelements stets in Richtung zum Axiallager kraftbeaufschlagt ist und somit stets in einer definierten Position befindlich ist. Da das Wandlerelement möglichst weit distalseitig, also unmittelbar vor der im Schaft befindlichen Optik angeordnet ist, verändert sich dieser für die Abbildungsschärfe wichtige Abstand auch bei wärmebedingten Längenänderungen innerhalb des Instrumentes praktisch nicht. Andererseits können sich Innenschaft und Schaft frei zueinander dehnen und bewegen, ohne dass dieser definierte Abstand, der durch die Anlage des Axiallagers stets gegeben ist, verändert wird.

Axiallager im Sinne der vorliegenden Erfindung ist ein Lager, welches Kräfte aufnimmt, die in Richtung der Längsachse des Schaftes auftreten. Dies schließt jedoch nicht aus, dass das Lager auch radial wirksam ist. Unter Schaft, Innenschaft oder Außenschaft im Sinne der vorliegenden Erfindung, werden rohrförmige Körper verstanden, wie sie typischerweise bei endoskopischen Instrumenten Verwendung finden und ineinander oder auch nebeneinander liegend angeordnet sind.

Die vorliegende Erfindung ist vorteilhaft bei Instrumenten mit Seitblickoptiken anwendbar, schließt deren Anwendung jedoch für andere Optiken nicht aus.

Um den Innenschaft über seine Länge präzise zu lagern, ist gemäß einer Weiterbildung der Erfindung vorgesehen, neben dem Axiallager ein weiteres Lager, vorzugsweise ein Radiallager vorzusehen, wobei das Axiallager wandlernah und das weitere Lager, also das Radiallager, nahe des Handhabenteils, vorzugsweise innerhalb des Handhabenteils angeordnet ist. Auf diese Weise ergibt sich für den Innenschaft auch bei großer Längsausdehnung eine präzise Führung innerhalb des Schaftes. Das Radiallager kann dabei in einfacher Form als Kugellager ausgebildet und so angeordnet sein, dass in diesem Bereich ein axialer Längenausgleich zwischen Innenschaft und Schaft erfolgen kann.

Das Federelement kann vorteilhaft durch eine Schraubenfeder gebildet sein, welche bevorzugt im Bereich des Handhabenteils angeordnet ist, den Innenschaft mit geringem Abstand umgibt und die einerseits sich an dem weiteren mit dem Innenschaft verbundenen Lager und andererseits innerhalb des Handhabenteils abstützt. Die Federkraft wirkt somit auf das Lager, welches fest mit dem Innenschaft verbunden ist und innerhalb des Handhabenteils in Längsrichtung des Schaftes verschiebbar geführt ist. Dabei ist die Schraubenfeder auf der dem Axiallager abgewandten Seite des weiteren Lagers angeordnet, so dass die Kraftwirkung stets in Richtung auf das Axiallager gerichtet ist.

Gemäß einer vorteilhaften Weiterbildung der Erfindung ist der Schaft des Instrumentes im Handhabenteil festgelegt, wobei der Innenschaft den Schaft in diesem Bereich proximalseitig überragt, d. h. deutlich weiter als der Schaft in den Handhabenteil hineinragt. Dies ist besonders günstig, da zum einen dann genügend Raum für die Anordnung des weiteren Lagers und der Feder verbleibt zum anderen weiterer Raum für die konstruktive Ausbildung der Betätigungsmittel zum Drehen des Innenschaftes gegenüber dem Instrument.

Das Axiallager selbst ist vorteilhaft durch zwei Lagerringe gebildet, von denen einer schaftseitig und der andere innenschaftseitig festgelegt ist. Die Lagerringe sind vorzugsweise aus einem keramischen Werkstoff gefertigt, so dass das Lager einerseits temperaturfest ist und andererseits schmierstofffrei sowie reibungs- und verschleißarm arbeitet.

Vorteilhaft ist der Innenschaft durch ein Zylinderrohr gebildet, welches sich vom Handhabenteil bis weit in den Schaft hinein erstreckt und das an seinem wandlernahen Ende mit einem den Wandler aufnehmenden Hohlkörper in Schaftrichtung längsverstellbar verbunden ist. Durch diese Anordnung können etwaige Fertigungstoleranzen ausgeglichen werden, insbesondere kann bei der Montage der Wandler zur Optik in Achsrichtung ausgerichtet und festgelegt werden.

Gemäß einer Weiterbildung der Erfindung ist der feststehende Lagerring des Axiallagers nicht unmittelbar am Schaft festgelegt, sondern an der dem Handhabenteil zugewandten Stirnseite einer Hülse, die ihrerseits fest, vorzugsweise durch Schweißen, mit dem Schaft verbunden ist.

Auch der drehbare Lagerring des Axiallagers ist vorteilhaft nicht unmittelbar mit dem Innenschaft verbunden, sondern ist auf der dem Handhabenteil abgewandten Stirnseite einer Buchse festgelegt, welche fest mit dem Innenschaft verbunden ist. Diese Buchse überragt das wandlemahe Ende des Innenschaftes und ist vorteilhaft mit diesem verschweißt. Dabei übergreift diese Buchse vorteilhaft nicht nur das wandlerseitige Ende des Innenschaftes, sondern auch das dem gegenüberliegende Ende des den Wandler aufnehmenden Hohlkörpers und weist darüber hinaus Mittel zum vorzugsweise lösbaren Festlegen des Hohlkörpers auf. Typischerweise ist in einer Querbohrung der Buchse eine Madenschraube eingelassen, welche nach dem axialen Ausrichten der Bauteile zueinander eingeschraubt wird und damit den Hohlkörper, welcher den Wandler trägt, gegenüber dem Innenschaft festlegt.

Um das Instrument hermetisch dicht abschließen zu können, also auch auf verschleißanfällige Dichtungen verzichten zu können, ist in einer Weiterbildung der Erfindung vorgesehen, die Kupplung zwischen Innenschaft einerseits und einem auf dem Handhabenteil drehbar angeordneten Stellring andererseits magnetisch vorzusehen. Eine solche Anordnung ist in DE 195 21 654 C2 im Einzelnen beschrieben, auf diese wird hiermit ausdrücklich Bezug genommen.

Das erfindungsgemäße Instrument kann bei entsprechender Ausbildung vollständig hermetisch abgeschlossen sein, und zwar im Prinzip dichtungsfrei. So kann beispielsweise das distale Ausblickfenster in an sich bekannter Weise durch Löten stoffschlüssig mit dem Schaft verbunden sein, ebenso wie der Handhabenteil durch Verwendung einer Magnetkupplung eine geschlossene metallische Hülle aufweisen kann. Ein Problem stellt jedoch häufig die Durchführung der Lichtleiter dar, da das Lichtleiterbündel, das von dem Lichtleiteranschluss am Handhabenteil bis zum distalen Ende des Schaftteils durch das Instrument geführt ist, an den Enden zwar in einem den Raum zwischen den Lichtleitern füllenden Kunststoff/Klebstoff eingebettet ist, dieser Stoff jedoch wie fast alle Kunststoffe nicht völlig dampfundurchlässig ist. Es kann also im Laufe der Zeit Dampf in diesen Bereich eindiffundieren, was unerwünscht ist.

Gemäß einer Weiterbildung der vorliegenden Erfindung ist daher vorgesehen, die Lichtleiter innerhalb des Handhabenteils innerhalb einer Lichtleiterführung anzuordnen, wobei diese Lichtleiterführung gegenüber dem Inneren der Handhabe druckdicht abgeschlossen ist. In Weiterbildung der Erfindung erfolgt dies in analoger Weise im Schaftbereich dadurch, dass die Lichtleiter im Bereich des Schaftes zwischen dem Schaft und einem diesen umgebenen Außenschaft geführt sind. Da der Schaft selbst typischerweise aus Metall, insbesondere Edelstahl besteht, ist dieser diffusionsdicht und ein Eintritt von Dampf auch auf diesem Wege ausgeschlossen.

Durch die diffusionsdichte Lichtleiterführung im Handhabenteil und im Schaftteil ist nun auch sichergestellt, dass sowohl Handhabenteil als auch Schaft vollständig diffusionsdicht gegenüber dem Raum ausgeführt sind, welcher das Lichtleiterbündel führt.

Um eine vollständige Diffusionsdichtheit zu erlangen, ist es erforderlich, die Lichtleiterführung metallisch, typischerweise aus Edelstahl auszubilden. Um etwaige Längendehnungen zuzulassen und um auch im Falle einer Reparatur ein gewisses Spiel zu haben, ist es zweckmäßig, die Lichtleiterführung zwischen dem eigentlichen Handhabenteil und dem Schaft zu unterbrechen und dort einen Freiraum vorzusehen, in welchem das Lichtleiterbündel sichelförmig verläuft und somit gewisse Längenänderungen - sei es temperaturbedingt oder auch reparaturbedingt - aufgefangen werden können.

Darüber hinaus ist es zweckmäßig, im Bereich des Handhabenteils in der Lichtleiterführung einen Faltenbalg vorzusehen, der insbesondere zur Kompensation von reparaturbedingten Längenänderungen vorgesehen ist, wenn beispielsweise der Handhabenteil zu Reparaturzwecken aufgeschnitten und nach erfolgter Reparatur wieder verschweißt wird, da sich dann die Länge des Handhabenteils ändern kann und somit auch eine Anpassung im Bereich der Lichtleiterführung erforderlich ist. Die übrige Lichtleiterführung ist vorteilhaft rohrförmig und aus Metall ausgebildet, an den Anschlussenden mit dem ebenfalls aus Metall bestehenden Faltenbalg verschweißt sowie endseitig durch Schweißen mit dem Handhabenteil verbunden.

Die Erfindung ist nachfolgend anhand eines an der Zeichnung dargestellten Ausführungsbeispiels näher erläutert. Es zeigen:
- Fig. 1: einen Längsschnitt durch ein endoskopisches Instrument gemäß der Erfindung,

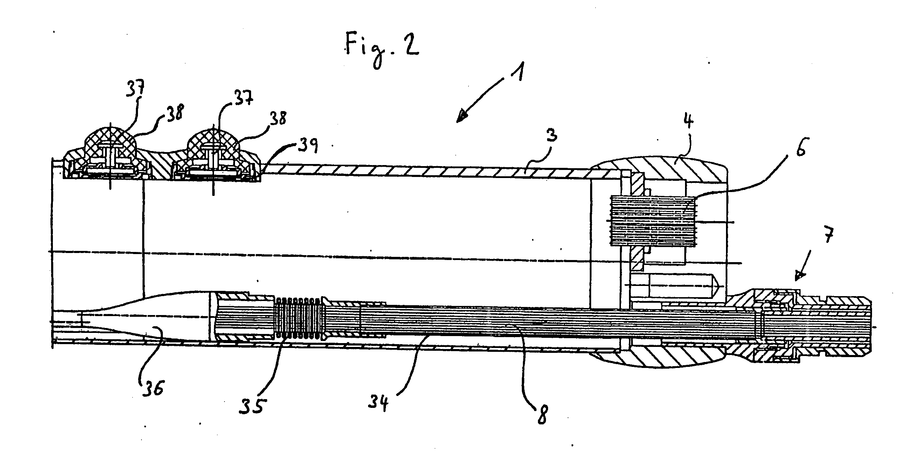

- Fig. 2: in vergrößerter Schnittdarstellung den proximalen Teil des Handhabenteils gemäß Fig. 1 in vereinfachter Darstellung,
- Fig. 3: in vergrößerter Darstellung den inneren distalen Teil des Handhabenteils gemäß Fig. 1,
- Fig. 4: in vergrößerter Darstellung einen Schaftabschnitt im Längsschnitt.

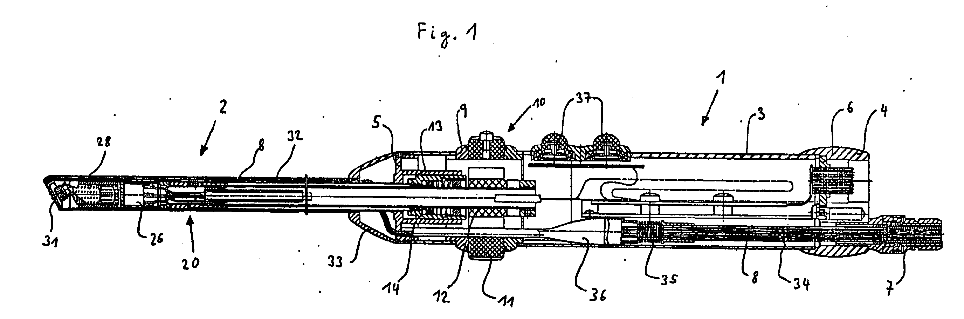

Bei dem anhand der Figuren dargestellten Instrument handelt es sich um ein Endoskop mit einem Handhabenteil 1 und einem Schaftteil 2. Der Schaftteil 2 ist in Fig. 1 verkürzt dargestellt und dient zum Einführen in eine natürliche oder künstlich geschaffene Körperhöhle. Der Handhabenteil 1 besteht im Wesentlichen aus einem zylindrischen Rohrabschnitt 3, welcher proximalseitig durch einen diesen übergreifenden Verschlussteil 4, der an den Rohrabschnitt 3 angeschweißt ist, dicht abgeschlossen ist. Distalseitig wird der Rohrabschnitt 3 durch ein ebenfalls durch Schweißen diffusionsdicht damit verbundenes distales Verschlussteil 5 abgeschlossen.

In dem proximalen Verschlussteil ist eine keramische Anschlussdurchführung 6, zum Herausführen sämtlicher elektrischer Leitungen vorgesehen, welches in das metallische Verschlussteil 4 eingelötet ist. Weiterhin ist in dem proximalen Verschlussteil 4 ein Lichtleitkabelanschlussstutzen 7 eingeschweißt, der in an sich bekannter Weise ausgebildet ist und im Inneren ein Bündel von Lichtleitfasern 8 trägt, dessen Zwischenräume mit Kunststoff bündig ausgefüllt sind.

Der zylindrische Rohrabschnitt 3 ist im distalseitigen Teil durch den feststehenden Teil 9 einer Magnetkupplung 10 unterbrochen. Ausbildung und Wirkweise dieser Magnetkupplung 10 ist in DE 195 21 654 C2 beschrieben, auf die insoweit verwiesen wird. Auf dem feststehenden Teil 9 ist ein Stellring 11 drehbar und mit Dichtungen gegenüber dem feststehenden Teil 9 abgedichtet gelagert. Die Dichtungen dichten nicht gegenüber dem Inneren des Handhabenteils 1 ab, sondern lediglich gegenüber dem zwischen Stellring 11 und feststehendem Teil 9 gebildeten Raum. Der Handhabenteil ist in diesem Bereich durch den feststehenden Teil 9 sowie den sich zu beiden Seiten daran anschließenden Rohrabschnitt 3 diffusionsdicht abgeschlossen. Der Stellring trägt an seiner Innenseite einen nicht im Einzelnen dargestellten Magneten, der mit einem ebenfalls nicht im Einzelnen dargestellten Magneten zusammenwirkt, welcher auf einem Innenschaft 12 in diesem Bereich angeordnet ist.

Dieser Innenschaft 12 ist drehbar innerhalb des Instrumentes gelagert. Hierzu weist der distale Verschlussteil 5 einen sich proximalwärts in das Handhabenteil 1 erstreckenden hohlzylindrischen Abschnitt 13 auf, welches fest mit einer Hülse 14 verbunden ist, welche distalseitig einen Schaft 15 trägt, welcher den Innenschaft 12 im gesamten Schaftteil 2 umgibt und der feststehend ist.

Weiterhin ist innerhalb der Hülse ein Kugellager 16 verschiebbar geführt, dessen Innenring distalseitig an der Stirnseite einer Innenhülse 17 anliegt, welche fest mit dem Innenschaft 12 verbunden ist. Innerhalb der Hülse 14 ist eine Schraubenfeder 18 angeordnet, die sich einerseits am Kugellager 16 und andererseits an einer Schraube 19 abstützt, die mit ihrem Außengewinde im Innengewinde der Hülse 14 festgelegt ist. Die Schraube 19 weist eine zentrale Durchgangsbohrung auf, durch welche der Innenschaft 12 bewegbar mit Spiel geführt ist.

Der Innenschaft 12, der im Handhabenteil 1 durch das Kugellager 16 radial geführt ist, reicht bis weit in den Schaftteil 2 hinein und ist dort in einem Axiallager 20 in Achsrichtung des Schaftteils 2 geführt, welches auch die ständig hierauf wirkenden Kräfte der Schraubenfeder 8 aufnimmt, die durch den Innenschaft 12 übertragen werden. Das Axiallager 20 ist durch zwei keramische Lagerringe 21 und 22 gebildet, von denen der mit dem Innenschaft 12 mitdrehende Lagerring 21 an der distalen Stirnseite einer Buchse 23 befestigt ist, welche das distale Ende des Innenschaftes 12 überragt und fest mit diesem verbunden ist. Der feststehende Lagerring 22 ist an der proximalen Stirnseite einer Hülse 24 befestigt, die fest mit dem Schaft 15 verbunden ist.

Innerhalb der Buchse 23 sowie des distalen Endes des Innenschaftes 12 ist ein rohrförmig, nach distalwärts abgestufter Hohlkörper 25 eingegliedert, welcher ein CCD-Element 26 trägt. Dieser Hohlkörper 25 ist mit Spiel innerhalb der Hülse 24 gelagert. Innerhalb der Buchse 23 ist der Hohlkörper 25 mittels einer dort in einer Querbohrung angeordneten Madenschraube 27 im Bezug auf den Innenschaft 12 festgelegt. Diese Madenschraube 27 wird erst eingedreht, wenn die Längsausrichtung des CCD-Elements 26 im Bezug auf die distalseitige Optik 28 erfolgt ist, so dass sichergestellt wird, dass das CCD-Element 26 stets an der vorgesehenen Stelle zur Erzeugung einer scharfen Abbildung angeordnet ist. Nach Eindrehen der Madenschraube 27 ist der Hohlkörper 25 im Bezug auf die Buchse 23 und den damit festverbundenen Innenschaft 12 festgelegt. Um die Madenschraube 27 zu erreichen ist eine Ausnehmung 29 im Schaft 15 vorgesehen. Um die Ausnehmung 29 zu verschließen ist ein weiterer Schaft 30 über den Schaft 15 gesteckt und distalseitig der Ausnehmung 29 mit diesem verschweißt ebenso wie proximalseitig. Das distale Ende des Schaftes 15 ist durch ein schräg zur Längsachse angeordnetes Fenster 31 abgeschlossen, das in den Schaft 15 diffusionsdicht eingelötet ist. Die dahinterliegende zugehörige Seitblickoptik 28 ist an sich bekannt und daher hier nicht im Einzelnen beschrieben.

Bei der vorbeschriebenen Konstruktion kann durch Drehen am Stellring 11 über die magnetische Kupplung 12 der Innenschaft 12 gegenüber dem Instrument gedreht werden, um so eine Nachführung des CCD-Elements 26 herbeizuführen, wenn das Instrument um seine Längsachse gedreht worden ist. Dabei werden die Axialkräfte stets durch das Axiallager 20 aufgenommen, wobei die Schraubenfeder 18 dafür sorgt, dass der Innenschaft 12 immer in Richtung zum distalen Instrumentenende kraftbeaufschlagt ist. So ist auch bei Erwärmungen sichergestellt, dass sich der Innenschaft 12 in Längsrichtung dehnen kann, andererseits jedoch der Abstand zwischen CCD-Element 26 und der Optik 28 praktisch unverändert bleibt.

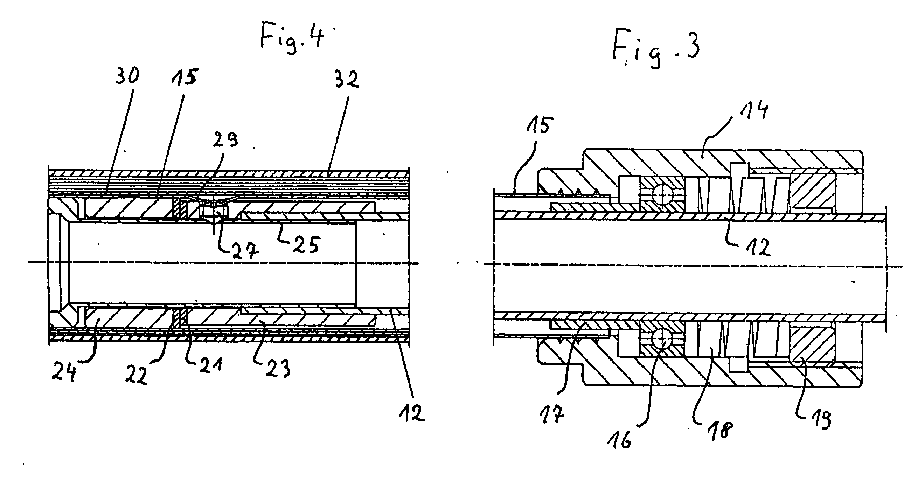

Die Lichtleitfasern sind im Bereich des Schaftteiles 2 zwischen dem Schaft 15 bzw. dem diesen überdeckenden Schaft 30 und einem Außenschaft 32 geführt, wobei der Außenschaft 32 exzentrisch zum Schaft 15 angeordnet ist, so dass sich in den Darstellungen gemäß Fig. 1 und 4 im Schaftteil 2 oben ein Freiraum für die Lichtleitfasern 8 ergibt. Die Lichtleitfasern 8 enden stirnseitig neben dem Fenster 31 jenseits des Schaftes 15, so dass der Schaft 15 gegenüber dem dort gebildeten Lichtleitfaserkanal diffusionsdicht abgeschlossen ist.

Das Handhabenteil 1 ist distalseitig durch eine Kappe 33 abgeschlossen, welche einerseits an dem distalen Verschlussteil 5 angeschweißt und andererseits durch Schweißen mit dem proximalen Ende des Außenschaftes 32 verbunden ist. Hierdurch ergibt sich innerhalb des durch die Kappe 33 umgebenden Raumes ein gegenüber dem Schaftteil 2 einerseits und gegenüber dem Handhabenteil 1 andererseits dicht abgeschlossener Raum, der lediglich zur Durchführung der Lichtleitfasern 8 dient und in welchem die Lichtleitfasern sichelförmig um den Schaft 15 herum geführt sind.

Innerhalb des distalen Verschlussteils 5 ist eine Ausnehmung vorgesehen, durch welche das Lichtleitfaserbündel 8 in Fig. 1 an der Unterseite des Handhabenteils 1 in das Innere desselben gelangt und dort etwa parallel zum Rohrabschnitt 3 an der Unterseite bis zum Lichtleitkabelanschlussstutzen 7 geführt ist. Innerhalb des Bereichs vom distalen Verschlussteil 5 bis zum proximalen Verschlussteil 4 sind die Lichtleitfasern 8 innerhalb einer Lichtleitfaserführung aus Metall geführt, so dass dieser Raum diffusionsdicht gegenüber dem Inneren des Handhabenteils 1 ausgebildet ist. Dabei erfolgt die Führung vom proximalen Verschlussteil 4 zunächst durch ein zylindrisches Führungsrohr 34, dann durch einen metallischen Faltenbalg 35 und schließlich durch ein Hüllstück 36, welches den im Querschnitt runden Faserstrang in einen abgeflachten über einen Teilkreis längs des Rohrabschnittes 3 erstreckenden und der Wölbung des Rohrabschnitts 3 angepassten Kanal überführt. Dieses zum Teil abgeflachte und an die Rundung des Rohrabschnitts 3 angepasste Hüllstückteil 36 dient dazu, die Lichtleitfasern 8 durch den Bereich der Magnetkupplung 10 zu führen, ohne die dort vorgegebenen Abstände zwischen den Magneten vergrößern zu müssen, also eine möglichst flache Durchführung zu bilden. Die Lichtleiterführung ist endseitig mit den jeweils anschließenden Verschlussteilen 4, 5 verschweißt.

An der Oberseite des Handhabenteils 1 sind zwei Drucktasten 37 angeordnet, die an ihrer Unterseite einen Magneten tragen und an ihrer Oberseite durch einen elastischen Kunststoff 38 überdeckt sind. Unterhalb der Magnete ist das Schaltergehäuse durch eine dünne Blechplatte 39 abgeschlossen, die diffusionsdicht mit dem Rohrabschnitt 3 durch Schweißen verbunden ist. Unterhalb dieser Blechplatte 39 ist eine Platine mit Hallelementen angeordnet, so dass durch die Blechplatte 39 hindurch ein Schaltvorgang mittels einer der Taster 37 ausgelöst werden kann. Die weitere Elektronik ist innerhalb des Handhabenteils 1 angeordnet. Die Schaltfunktion der Drucktasten 37 ist frei programmierbar, beispielsweise zum Auslösen des Weißabgleichs der Videokamera oder anderer Funktionen.

Zu Reparaturzwecken kann das Handhabenteil 1 geöffnet werden, indem der Rohrabschnitt 3 beispielsweise im Bereich zwischen dem Schalter 37 und dem feststehenden Teil 9 aufgetrennt wird. Um die dann getrennten Gehäusehälften so weit auseinander ziehen zu können, dass die darin befindlichen Bauteile, sei es die Elektronik oder auch die schaftseitigen Bauteile erreichen zu können, ist es erforderlich die Schafthälften auseinander zu ziehen. Hierzu ist der Faltenbalg 35 erforderlich, der es ermöglicht, die Bauteile ohne Auftrennung der Lichtleitfaserführung auseinander ziehen zu können. Der Faltenbalg 35 dient darüber hinaus auch beim späteren Verschließen des Handhabenteils 1 durch umlaufende Schweißung dazu, etwaige Längenänderungen des Handhabenteils 1 auszugleichen.

Das Lichtleiterbündel 8 ist also innerhalb des Handhabenteils 1 durch die Lichtleiterführung diffusionsdicht gegenüber dem übrigen Inneren des Handhabenteils 1 abgeschlossen. Lediglich im Bereich der Kappe 33 liegen die Lichtleiter 8 frei im Raum, allerdings ist dieser Raum sowohl gegenüber dem Handhabenteil als auch gegenüber dem Schaft 15 bzw. 30 diffusionsdicht abgeschlossen. Innerhalb des Schaftteils ist das Lichtleitfaserbündel 8 exzentrisch zwischen dem Schaft 15 bzw. 30 und dem Außenschaft 32 geführt, so dass das eigentliche endoskopische Instrument gegenüber dem Lichtleitfaserbündel 8 sowie gegenüber der Außenatmosphäre hermetisch und diffusionsdicht abgeschlossen ist. Sämtliche Verbindungen sind stoffschlüssig durch Löten oder Schweißen gebildet.

### Bezugszeichenliste

- 1: Handhabenteil
- 2: Schaftteil
- 3: Rohrabschnitt
- 4: proximales Verschlussteil
- 5: distales Verschlussteil
- 6: Anschlussdurchführung
- 7: Lichtleitkabelanschlussstutzen
- 8: Lichtleitfasern
- 9: feststehendes Teil
- 10: Magnetkupplung
- 11: Stellring
- 12: Innenschaft
- 13: Hohlzylinder
- 14: Hülse
- 15: Schaft
- 16: Kugellager
- 17: Innenhülse
- 18: Schraubenfeder
- 19: Schraube
- 20: Axiallager
- 21: Lagerring drehend
- 22: Lagerring fest
- 23: Buchse
- 24: Hülse
- 25: Hohlkörper
- 26: CCD-Element
- 27: Madenschraube
- 28: Optik
- 29: Ausnehmung
- 30: Schaft
- 31: Fenster
- 32: Außenschaft
- 33: Kappe
- 34: Führungsrohr
- 35: Faltenbalg
- 36: Hüllstück
- 37: Drucktasten
- 38: Kunststoffumhüllung
- 39: Blechplatte

## Patentansprüche

1. Optisches Instrument, insbesondere Endoskop oder Technoskop, mit einem Schaft (15), mit einem nahe dem freien Schaftende angeordneten Ausblickfenster (31), mit einem Handhabenteil (1) am anderen Ende des Schaftes (15), mit einem innerhalb des Schaftes (15) angeordneten optoelektrischen Wandler (26), der drehbar innerhalb des Schaftes (15) angeordnet und an einem drehbar innerhalb des Schaftes (15) gelagerten Innenschaft (12) angebracht ist, mit Betätigungsmitteln (10) zum Drehen des Wandlers (26) im Schaft (15) und mit mindestens einem Federelement (18), **dadurch gekennzeichnet, dass** zwischen Schaft (15) und Innenschaft (12) ein Axiallager (20) angeordnet ist, und dass das Federelement (18) den Innenschaft (12) in Richtung zum Axiallager (20) kraftbeaufschlagt.

2. Instrument nach Anspruch 1, **dadurch gekennzeichnet, dass** der Innenschaft (12) mittels eines weiteren Lagers (16), vorzugsweise eines Radiallagers, insbesondere eines Kugellagers drehbar innerhalb des Instrumentes (1,2) gelagert ist, wobei das Axiallager (20) wandlernah und das weitere Lager (16) vorzugsweise innerhalb des Handhabenteils (1) angeordnet ist.

3. Instrument nach einem der vorhergehenden Ansprüche, **dadurch gekennzeichnet, dass** das Federelement (18) durch eine den Innenschaft (12) umgebende Schraubenfeder (18) gebildet ist, die sich innenschaftseitig an dem weiteren Lager (16) sowie innerhalb des Handhabenteils (1) abstützt.

4. Instrument nach einem der vorhergehenden Ansprüche, **dadurch gekennzeichnet, dass** der Schaft (15) im Handhabenteil (1) festgelegt und in diesem Bereich vom Innenschaft (12) proximalseitig überragt ist.

5. Instrument nach einem der vorhergehenden Ansprüche, **dadurch gekennzeichnet, dass** das Axiallager (20) durch zwei vorzugsweise aus Keramik bestehende Lagerringe (21, 22) gebildet ist.

6. Instrument nach einem der vorhergehenden Ansprüche, **dadurch gekennzeichnet, dass** der Innenschaft (12) durch ein vom Schaft (15) bis zum Handhabenteil (1) sich erstreckendes Zylinderrohr gebildet ist, das an seinem wandlernahen Ende mit einem den Wandler aufnehmenden Hohlkörper (25) in Schaftrichtung längsverstellbar verbunden ist.

7. Instrument nach einem der vorhergehenden Ansprüche, **dadurch gekennzeichnet, dass** der feststehende Lagerring (22) des Axiallagers (20) auf der dem Handhabenteil zugewandten Stirnseite einer Hülse (24) festgelegt ist, welche fest, vorzugsweise durch Schweißen, mit dem Schaft (15) verbunden ist.

8. Instrument nach einem der vorhergehenden Ansprüche, **dadurch gekennzeichnet, dass** der drehbare Lagerring (21) des Axiallagers (20) auf der dem Handhabenteil abgewandten Stirnseite einer Buchse (23) festgelegt ist, welche fest mit dem Innenschaft (12) verbunden ist.

9. Instrument nach einem der vorhergehenden Ansprüche, **dadurch gekennzeichnet, dass** die Buchse (23) das wandlerseitige Ende des Innenschaftes (12) sowie das gegenüberliegende Ende des den Wandler (26) aufnehmenden Hohlkörpers (25) übergreift, wobei in der Buchse (23) Mittel (27) zum vorzugsweise lösbaren Festlegen des Hohlkörpers (25) an der Buchse (23) vorgesehen sind.

10. Instrument nach einem der vorhergehenden Ansprüche, **dadurch gekennzeichnet, dass** der Innenschaft (12) magnetisch mit einem auf dem Handhabenteil (1) drehbar angeordnetem Stellring (11) gekuppelt ist.

11. Instrument nach einem der vorhergehenden Ansprüche, **dadurch gekennzeichnet, dass** Lichtleiter (8) durch das Instrument (1, 2) geführt sind, wobei mindestens im Bereich des Handhabenteils (1) eine Lichtleiterführung (34, 35, 36) vorgesehen ist, welche den Raum, in welchem die Lichtleiter (8) durch das Handhabenteil (1) geführt sind, gegenüber dem Inneren des Handhabenteils (1) diffusionsdicht abschließt.

12. Instrument nach einem der vorhergehenden Ansprüche, **dadurch gekennzeichnet, dass** die Lichtleiterführung (34, 35, 36) innerhalb des Handhabenteils (1) einen Faltenbalg (35) zur Kompensation von insbesondere reparaturbedingten Längenänderungen aufweist.

13. Instrument nach einem der vorhergehenden Ansprüche, **dadurch gekennzeichnet, dass** die Lichtleiterführung (34, 35, 36) rohrförmig und aus Metall gebildet ist und an beiden Enden vorzugsweise durch Schweißen mit dem Handhabenteil (1) verbunden ist.

14. Instrument nach einem der vorhergehenden Ansprüche, **dadurch gekennzeichnet, dass** die Lichtleiter (8) im Bereich des Schaftteiles (2) zwischen dem diffusionsdichten Schaft (15) und einem diesen umgebenden Außenschaft (32) geführt sind.

## Claims

1. An optical instrument, in particular an endoscope or technoscope, with a shank (15), with a viewing window (31) arranged close to the free shank end, with a handle part (1) on the other end of the shank (15), with an optoelectrical transducer (26) which is arranged within the shank (15), is rotatably arranged within the shank (15) and is attached on an inner shank (12) rotatably mounted within the shank (15), with actuation means (10) for rotating the transducer (26) in the shank (15) and with at least one spring element (18), **characterised in that** a thrust bearing (20) is arranged between the shank (15) and the inner shank (12), and that the spring element (18) impinges the inner shank (12) with force in the direction of the thrust bearing (20).

2. An instrument according to claim 1, **characterised in that** the inner shank (12) is mounted in a rotatable manner within the instrument (1, 2) by way of a further bearing (16), preferably a radial bearing, in particular a ball bearing, wherein the thrust bearing (20) is arranged close to the transducer, and the second bearing (16) is arranged preferably within the handle part (1).

3. An instrument according to one of the preceding claims, **characterised in that** the spring element (18) is formed by a helical spring (18) which surrounds the inner shank (12) and which is supported on the inner shank side on a further bearing (16), as well as within the handle part (1).

4. An instrument according to one of the preceding claims, **characterised in that** the shank (15) is fixed in the handle part (1), and the inner shank (12) projects beyond it on the proximal side, in this region.

5. An instrument according to one of the preceding claims, **characterised in that** the thrust bearing (20) is formed by two bearing rings (21, 22) which preferably consist of ceramic.

6. An instrument according to one of the preceding claims, **characterised in that** the inner shank (12) is formed by a cylinder tube which extends from the shank (15) up to the handle part (1) and which, at its end close to the transducer, is connected in a longitudinally adjustable manner in the shank direction, to a hollow body (25) receiving the transducer.

7. An instrument according to one of the preceding claims, **characterised in that** the stationary bearing ring (22) of the thrust bearing (20) is fixed on the end-side of a sleeve (24), said end-side facing the handle part, wherein the sleeve is connected to the shank (15) in a fixed manner, preferably by way of welding.

8. An instrument according to one of the preceding claims, **characterised in that** the rotatable bearing ring (21) of the thrust bearing (20) is fixed on the end-side of a bush (23), said end-side being away from the handle part, wherein the bush (23) is connected to the instrument shank (12) in a fixed manner.

9. An instrument according to one of the preceding claims, **characterised in that** the bush (23) engages over the transducer-side end of the inner shank (12) as well as the opposite end of the hollow body (25) receiving the transducer (26), wherein means (27) for the preferably releasable fastening of the hollow body (25) on the bush (23), are provided in the bush (23).

10. An instrument according to one of the preceding claims, **characterised in that** the inner shank (12) is magnetically coupled to an adjustment ring (11) rotatably arranged on the handle part (1).

11. An instrument according to one of the preceding claims, **characterised in that** fibre optics (8) are led through the instrument (1, 2), wherein a fibre optic guide (34, 35, 36) is provided at least in the region of the handle part (1) and closes off the space in which the fibre optics (8) are led through the handle part (1), with respect to the inside of the handle part (1).

12. An instrument according to one of the preceding claims, **characterised in that** the fibre optic guide (34, 35, 36), within the handle part (1), comprises a bellows (35) for compensating length changes which in particular are due to repairs.

13. An instrument according to one of the preceding claims, **characterised in that** the fibre-optic guide (34, 35, 36) is tubular and is formed of metal and is connected at both ends, preferably by way of welding, to the handle part (1).

14. An instrument according to one of the preceding claims, **characterised in that** the fibre-optics (8) are led in the region of the shank part (2), between the diffusion-tight shank (15) and an outer shank (32) surrounding this.

## Revendications

1. Instrument d'optique, en particulier endoscope ou technoscope, comportant une tige (15), une fenêtre d'observation (31) disposée près de l'extrémité libre de la tige, une partie poignée (1) en l'autre extrémité de la tige (15), un convertisseur opto-électrique (26) disposé à l'intérieur de la tige (15), qui est disposé mobile en rotation à l'intérieur de la tige (15) et est solidaire d'une tige intérieure (12) disposée mobile en rotation à l'intérieur de la tige (15), des moyens d'actionnement (10) servant à faire tourner le convertisseur (26) dans la tige (15), et au moins un élément élastique (18), **caractérisé en ce qu'**un palier axial (20) est disposé entre la tige (15) et la tige intérieure (12) et **en ce que** l'élément élastique (18) sollicite la tige intérieure (12) avec une force en direction du palier axial (20).

2. Instrument selon la revendication 1, **caractérisé en ce que** la tige intérieure (12) est montée mobile en rotation à l'intérieur de l'instrument (1, 2), au moyen d'un autre palier (16), de préférence d'un palier radial, en particulier d'un roulement à billes, le palier axial (20) étant disposé près du convertisseur et l'autre palier (16) étant disposé de préférence à l'intérieur de la partie poignée (1).

3. Instrument selon l'une des revendications précédentes, **caractérisé en ce que** l'élément élastique (18) est formé d'un ressort hélicoïdal (18) entourant la tige intérieure (12), qui prend appui contre l'autre palier (16) côté tige intérieure ainsi qu'à l'intérieur de la partie poignée (1).

4. Instrument selon l'une des revendications précédentes, **caractérisé en ce que** la tige (15) est fixée dans la partie poignée (1) et, dans cette région, se prolonge au-delà de la tige intérieure (12) sur le côté proximal.

5. Instrument selon l'une des revendications précédentes, **caractérisé en ce que** le palier axial (20) est formé de deux bagues d'appui (21, 22) de préférence en céramique.

6. Instrument selon l'une des revendications précédentes, **caractérisé en ce que** la tige intérieure (12) est formée d'un tube cylindrique qui s'étend depuis la tige (15) jusqu'à la partie poignée (1), et qui, en son extrémité proche du convertisseur, est relié, de façon réglable en longueur dans le sens de la tige, à un corps creux (25) qui reçoit le convertisseur.

7. Instrument selon l'une des revendications précédentes, **caractérisé en ce que** la bague de roulement fixe (22) du palier axial (20) est fixée sur le côté frontal dirigé vers la partie poignée d'une douille (24) qui est reliée rigidement à la tige (15), de préférence par soudage.

8. Instrument selon l'une des revendications précédentes, **caractérisé en ce que** la bague de roulement pivotante (21) du palier axial (20) est fixée sur le côté frontal éloigné de la partie poignée d'un manchon (23) qui est relié rigidement à la tige intérieure (12).

9. Instrument selon l'une des revendications précédentes, **caractérisé en ce que** le manchon (23) recouvre l'extrémité côté convertisseur de la tige intérieure (12), ainsi que l'extrémité opposée du corps creux (25) qui reçoit le convertisseur (26), des moyens (27) étant prévus dans le manchon (23) pour la fixation, de préférence amovible, du corps creux (25) au manchon (23).

10. Instrument selon l'une des revendications précédentes, **caractérisé en ce que** la tige intérieure (12) est accouplée magnétiquement à une bague de réglage (11) qui est disposée mobile en rotation sur la partie poignée (1).

11. Instrument selon l'une des revendications précédentes, **caractérisé en ce que** des conducteurs de lumière (8) sont guidés à travers l'instrument (1, 2), cependant qu'il est prévu, au moins dans la région de la partie poignée (1), un guide de conducteurs de lumière (34, 35, 36) qui isole de façon étanche à la diffusion l'espace dans lequel les conducteurs de lumière (8) sont guidés à travers la partie poignée (1) par rapport à l'intérieur de la partie poignée (1).

12. Instrument selon l'une des revendications précédentes, **caractérisé en ce que** le guide de conducteurs de lumière (34, 35, 36) présente à l'intérieur de la partie poignée (1) un soufflet plissé (35) pour compenser les variations de longueur résultant en particulier de réparations.

13. Instrument selon l'une des revendications précédentes, **caractérisé en ce que** le guide de conducteurs de lumière (34, 35, 36) est tubulaire et métallique, et est relié à la partie poignée (1) en ses deux extrémités, de préférence par soudage.

14. Instrument selon l'une des revendications précédentes, **caractérisé en ce que**, dans la région de la partie tige (2), les conducteurs de lumière (8) sont guidés entre la tige (15) étanche à la diffusion et une tige extérieure (32) qui entoure celle-ci.
